# EUROPÄISCHE PATENTANMELDUNG

(11) **EP 3 156 083 A1**
(43) Veröffentlichungstag der Anmeldung: **19.04.2017**
(21) Anmeldenummer: 16187742.8
(22) Anmeldetag: 08.09.2016
(51) Int. Cl.: A61L 27/36, A61F 2/24

(54) **VERFAHREN ZUR DICKENKONTROLLE UND DREIDIMENSIONALEN FORMGEBUNG VON BIOLOGISCHEM GEWEBE WÄHREND DER FIXIERUNG**

(30) Priorität: 12.10.2015 DE 102015117318
(71) Anmelder: Biotronik AG, 8180 Bülach (CH)
(72) Erfinder: Rzany, Alexander, 90449 Nürnberg (DE); Erdbrügger, Wilhelm, 78467 Konstanz (DE); Hensel, Bernhard, 91052 Erlangen (DE)
(74) Vertreter: Randoll, Sören

(57) **Zusammenfassung**

Die Erfindung betrifft ein Verfahren zum Aufprägen einer 3D-Form auf ein biologisches Gewebe, insbesondere Perikardgewebe, während der Vernetzung des Gewebes, unter Verwendung eines Formwerkzeuges, das eine erste 3D-Anlagefläche zur flächigen Anlage an einer Oberseite des Gewebes sowie eine zweite 3D-Anlagefläche zur flächigen Anlage an einer Unterseite des Gewebes aufweist, wobei das Gewebe zwischen den beiden Anlageflächen angeordnet wird, so dass es beidseitig an diesen anliegt und dabei mittels eines Vernetzungsmittels vernetzt wird, so dass das vernetzte Gewebe nach Entnahme aus dem Formwerkzeug eine 3D-Form aufweist. Weiterhin betrifft die Erfindung ein Implantat mit einem solchen Gewebe.

## Beschreibung

Die Erfindung betrifft ein Verfahren zum Aufprägen einer 3D-Form auf ein biologisches Gewebe sowie ein Implantat, insbesondere in Form einer Herzklappenprothese, bei der ein solches Gewebe verwendet wird, insbesondere als Herzklappe.

Für die Dickenkontrolle von Gewebe während der Fixierung sind nach dem aktuellen Stand der Technik bereits Lösungen bekannt, die auf eine Fixierung der Gewebe zwischen porösen Keramikträgern abzielen (US20130310929A1).

Bei der Implantation von TAVI-Herzklappen (TAVI für Transkatheter-Aortenklappenimplantation bzw. engl. transcatheter aortic valve implantation) ist der Durchmesser des Kathetersystems, das die Herzklappe trägt, eine der entscheidenden Eigenschaften um kardiovaskuläre Komplikationen zu vermeiden bzw. einen derartigen Eingriff überhaupt durchführen zu können. Der Durchmesser der Katheterkapsel ist dabei der limitierende Faktor und hängt wesentlich von der Größe des Klappenstents und der Dicke des verwendeten Gewebes ab. Eine Dickenreduktion des Gewebes führt deshalb zu einem geringeren Raumbedarf und damit zu geringeren Kapseldurchmessern, insbesondere da das Gewebe bei der gecrimpten Klappe in Falten liegt.

Aktuelle Designs minimalinvasiv implantierbarer Herzklappen bestehen aus einem metallischen Klappenstent und mehreren Teilen biologischem Klappengewebe, welche die Klappenflügel (Leaflet) und die Abdichtung (Skirt) bilden. Um das biologische Gewebe mit dem Klappenstent mechanisch stabil zu verbinden, werden die Gewebestücke in Handarbeit einzeln mit dem Klappenstent und untereinander vernäht. Das Vernähen erfolgt dabei nicht kontinuierlich in Nähten mit einem Faden, sondern mit einzelnen Knoten. Je nach Klappendesign sind für eine stabile Verbindung der einzelnen Komponenten mehrere Hundert einzelner Knoten erforderlich. Diese werden in aufwändiger manueller Arbeit mit Nadel, Faden und Schere unter mikroskopischer Kontrolle angebracht. Aufgrund des aufwändigen Nahtprozesses ist ein hoher Ausschuss in einem relativ späten Teil der Prozesskette mit hochwertigen Ausgangskomponenten und hohem Personalaufwand praktisch nicht zu vermeiden. Weiterhin hat diese Art der Herstellung offensichtliche Nachteile hinsichtlich extremem personellen Zeitaufwand und schwieriger Skalierbarkeit der Prozesse hinsichtlich Stückzahlen. Zudem ist die Naht eine potentielle Schwachstelle hinsichtlich der mechanischen Wechselbelastung über die Lebensdauer des Klappenimplantates.

Hiervon ausgehend liegt der vorliegenden Erfindung die Aufgabe zugrunde, ein Verfahren zur Behandlung eines biologischen Gewebes sowie ein das Gewebe verwendendes Implantat zu schaffen, bei dem die vorgenannten Nachteile zumindest teilweise gemindert sind.

Diese Aufgabe wird durch ein Verfahren mit den Merkmalen des Anspruchs 1 sowie durch ein Implantat mit den Merkmalen des Anspruchs 9 gelöst. Vorteilhafte Ausgestaltungen dieser beiden Aspekte der Erfindung sind in den entsprechenden Unteransprüchen angegeben und werden nachfolgend beschrieben.

Gemäß Anspruch 1 ist erfindungsgemäß ein Verfahren zum Aufprägen einer 3D-Form auf ein biologisches Gewebe, insbesondere Perikardgewebe und bevorzugt kollagenreiches Perikardgewebe, während der Vernetzung des Gewebes unter Verwendung eines Formwerkzeuges vorgesehen, das eine erste 3D-Anlagefläche zur flächigen Anlage an einer Oberseite des Gewebes sowie eine zweite 3D-Anlagefläche zur flächigen Anlage an einer Unterseite des Gewebes aufweist, wobei das Gewebe zwischen den beiden Anlageflächen angeordnet bzw. eingespannt oder gepresst wird, so dass es beidseitig eng an diesen Anlageflächen anliegt und dabei mittels eines Vernetzungsmittels vernetzt wird, so dass das vernetzte Gewebe nach Entnahme aus dem Formwerkzeug eine 3D-Form aufweist.

Eine 3D-Anlagefläche ist im Sinne der vorliegenden Erfindung insbesondere eine nicht plane Anlagefläche, die also eine Krümmung im dreidimensionalen Raum aufweist. Insbesondere kann die zweite 3D-Anlagefläche einer Krümmung der ersten 3D-Anlagefläche folgen, so dass ein Gewebe mit 3D-Form in konstanter Dicke resultiert. Die beiden 3D-Anlageflächen können jedoch auch lokal oder grundsätzlich eine unterschiedliche Krümmung bzw. Verlauf aufweisen, so dass die Dicke des vernetzten Gewebes lokal eingestellt bzw. kontrolliert werden kann.

Die Erfindung ermöglicht somit mit Vorteil eine dreidimensionale Formgebung und Dickenreduktion bzw. -kontrolle von biologischen Geweben, insbesondere für Implantate, Herzklappenprothesen, vorzugsweise TAVI Herzklappenprothesen, mit dem Ziel eines geringeren Katheterdurchmessers, der Möglichkeit neuer Klappendesigns durch dreidimensionale Formgebung von Gewebestücken und der Reduktion von benötigten Nähten zwischen biologischem Gewebe und Stent zur Verwendung in Herzklappen und anderen Implantaten. Insbesondere können mit dem erfindungsgemäßen Verfahren Herzklappen aus Gewebe in einem Stück, also einstückig hergestellt werden.

Gemäß einer bevorzugten Ausführungsform des erfindungsgemäßen Verfahrens ist vorgesehen, dass die Dicke des vernetzten Gewebes durch einen Abstand der beiden Anlageflächen zueinander gesteuert bzw. auf einem maximalen Wert begrenzt wird. "Auf einen maximalen Wert begrenzt" bedeutet, dass der Abstand zwischen den Anlageflächen die größtmögliche Ausdehnung der Dicke des vernetzten Gewebes darstellt. Dies ermöglicht zusätzlich zur Formgebung die Dickenkontrolle des Gewebes.

Gemäß einer bevorzugten Ausführungsform des erfindungsgemäßen Verfahrens ist vorgesehen, dass das Formwerkzeug einen oberen vorzugsweise starren Formbereich aufweist, der z.B. aus einem porösen Material besteht, das jene erste 3D-Anlagefläche ausbildet, und dass das Formwerkzeug einen unteren Formbereich aufweist, der z.B. aus einem porösen Material besteht, das jene zweite 3D-Anlagefläche ausbildet. In einer bevorzugten Ausführungsform werden die Formbereiche vor der Vernetzung mit wässriger NaCl (vorzugsweise 0,9%w/v) behandelt, vorzugsweise durch Eintauchen der Formbereich in eine solche Lösung. Vorzugsweise sind die Formbereiche mit NaCl (vorzugsweise 0,9%w/v) getränkt.

Die Formbereiche sind also bevorzugt für das Vernetzungsmittel durchlässig, so dass dieses während der Vernetzung über die Formbereiche zu dem Gewebe gelangen und dieses kontaktieren kann.

Die vorzugsweise porösen Formbereiche bzw. Träger können eine gewünschte bzw. beliebige dreidimensionale Form (3D-Anlageflächen) aufweisen, wie sie z.B. durch 3D-Drucker hergestellt werden können. Die Formbereiche können z.B. durch offenporige Polymerträger gebildet sein, die selbst nicht mit den vernetzenden Agentien reagieren (z.B. poröses Polycarbonat). Andere geeignete (poröse) Formbereichs- bzw. Trägermaterialien sind z.B. gesintertes Borosilikatglas, Keramiken oder poröse Metalle. Porengröße, Porenverteilung und mechanische Eigenschaften des Trägers bestimmen dabei die Zufuhr des Vernetzungsmittels.

Vorzugsweise wird das zu vernetzende Gewebe im nativen Zustand zwischen die porösen bzw. durchlässigen Formbereiche bzw. Träger eingelegt und z.B. mit Hilfe einer Fixierung (insbesondere durch Klammern oder Schrauben) eng anliegend fixiert. Dadurch werden die Faserproteine im Gewebe in der räumlichen Anordnung stabilisiert, raumfordernde Strukturänderungen verhindert und die besagte dreidimensionale Form aufgeprägt.

Bei der Vernetzung des Gewebes werden die Kollagenfasern im Perikardgewebe mittels eines geeigneten Vernetzungsmittels durch den Einbau chemischer Bindungen quervernetzt. Das Vernetzungsmittel bindet an freie Aminogruppen der Kollagenfasern an und bildet chemisch stabile Verbindungen zwischen Kollagenfasern aus. So entsteht aus den dreidimensional angeordneten Kollagenfasern ein langzeitstabiles biologisches Material, welches zudem nicht mehr als biologisches Fremdmaterial erkannt wird. Durch die dreidimensionale Vernetzung bzw. Verknüpfung der einzelnen Kollagenfasern über das Vernetzungsmittel werden die Stabilität und die Beanspruchbarkeit des Gewebes deutlich erhöht. Dies ist insbesondere bei einem Einsatz als Gewebe einer Herzklappe entscheidend, wo das Gewebe etwa im Sekundentakt als Klappe öffnen und schließen soll.

Durch die zusätzliche dreidimensionale Formgebung des Gewebes während der Vernetzung (z.B. mit Glutaraldehyd) wird es möglich, das Gewebe bzw. den biologischen Teil des Klappenimplantates aus einer geringeren Anzahl an Gewebestücken durch Zusammennähen mit dem Klappenstent herzustellen. Dies ist insbesondere vorteilhaft, da das Vernähen von Formstücken sehr zeit- und damit kostenintensiv ist. Durch das vorgeschlagene Verfahren wird es möglich Klappenimplantate zeit- und kostenschonend herzustellen.

Für das vorgeschlagene Verfahren können grundsätzlich alle Arten von Gewebe vom Säugetier, einschließlich vom Menschen, verwendet werden. Bevorzugt ist nicht-humanes Gewebe. Insbesondere eignen sich solche Gewebe, die als Klappenmaterial in einer Herzklappe verwendet werden können. Bevorzugt sind dabei Perikardgewebe, insbesondere kollagenreiches Perikardgewebe, und Herzklappen, aber auch Haut, Bandgewebe, Sehnen, Bauchfell, Dura mater, Tela submucosa, insbesondere des Magen-Darm-Traktes, oder Rippenfell. Bei Herzklappen können alle Klappen verwendet werden, also Aorten-, Pulmonal-, Mitral- und Trikuspidalklappen. Des Weiteren sind bevorzugt Perikardgewebe vom Schwein, Schaf, Ziege, Pferd, Krokodil, Känguru, Strauß und vom Rind.

Im Rahmen dieser Anmeldung bezieht sich die Angabe %v/v auf einen Volumenprozentanteil. Wird bezüglich einer Lösung nichts anderes angegeben, so werden für die Lösungen hierin Wasser als Lösungsmittel verwendet. Eine 100 ml Lösung mit 5%v/v Glutaraldehyd enthält entsprechend 5 ml reines Glutaraldehyd (vorzugsweise mit einem Verhältnis der Absorptionen 235nm:280nm < 0.5). Die Angabe %w/v bezieht sich im Rahmen dieser Anmeldung auf einen Gewichtsanteil. 100 ml Lösung mit 0,9%w/v Natriumchlorid enthält entsprechend 0,9 g Natriumchlorid.

Gemäß einer Ausführungsform des erfindungsgemäßen Verfahrens handelt es sich bei dem Vernetzungsmittel um eine Glutaraldehyd-haltige Lösung, die vorzugsweise 0,01%v/v bis 2%v/v Glutaraldehyd aufweist, vorzugsweise in DPBS ohne Ca/Mg. Andere wässrige, dem Fachmann bekannte Puffer ohne Ca/Mg sind alternativ verwendbar. Ferner können andere Lösungen verwendet werden, die ein Vernetzungsmittel enthalten ausgewählt aus der Gruppe umfassend oder bestehend aus Glutaraldehyd, Carbodiimid, Formaldehyd, Glutaraldehyd-Acetale, Acyl-Azide, Cyanimid, Genepin, Tannin, Pentagalloyl-Glukose, Phytat, Proanthocyanidin, Reuterin und Epoxidverbindungen.

Gemäß einer Ausführungsform des erfindungsgemäßen Verfahrens ist weiterhin vorgesehen, dass das im Formwerkzeug angeordnete bzw. darin eingespannte Gewebe dem Vernetzungsmittel 1 bis 3 Tage, vorzugsweise 2 Tage, bei 2°C bis 10°C, vorzugsweise bei 4°C, ausgesetzt wird.

Gemäß einer Ausführungsform des erfindungsgemäßen Verfahrens ist weiterhin vorgesehen, dass das Gewebe anschließend dem Vernetzungsmittel 10 bis 18 Tage, vorzugsweise 14 Tage, vorzugsweise bei Raumtemperatur (typisch 20°C bis 25°C), ausgesetzt wird, wobei bevorzugt das Vernetzungsmittel alle 1 bis 3 Tage, vorzugsweise alle 2 Tage, ausgewechselt wird.

Gemäß einem weiteren Aspekt der Erfindung wird ein Implantat offenbart, das ein Gewebe aufweist, welchem eine 3D-Form mit einem erfindungsgemäßen Verfahren aufgeprägt wurde.

Gemäß einer bevorzugten Ausführungsform des Implantats ist das Implantat eine Herzklappenprothese, welche eine künstliche Herzklappe aus dem Gewebe aufweist, welches an einen expandierbaren oder selbstexpandierenden und per Katheter implantierbaren Grundkörper befestigt, bevorzugt vernäht, ist. Weiterhin ist auch eine Anwendung für Venenklappen, Prothesen oder vaskuläre Implantate denkbar.

Die Möglichkeit, die Dicke von biologischen Geweben, wie z.B. Perikardgeweben, während der Vernetzung mit Glutaraldehyd und deren dreidimensionale Form zu kontrollieren ermöglicht es, dünnere Gewebe für TAVI-Systeme bereitzustellen, die definierte dreidimensionale Formen haben. Dies hat nicht zuletzt zur Folge, dass Katheter mit geringeren Gesamtdurchmessern hergestellt werden können, die wiederum zu geringeren vaskulären Problemen bei der Einführung und Positionierung der TAVI-Herzklappen beitragen.

Ein weiterer großer Vorteil der erfindungsgemäßen Lösung ist die problemlose Integration der Dickenkontrolle durch mechanische Raumbeschränkung in bestehende Produktionsprozesse. Die vorgeschlagene Möglichkeit zum beliebigen Formen von dreidimensionalen porösen Trägern bzw. Formbereichen gelingt zunächst ohne den Einsatz neuer chemischer Substanzen. Dadurch kann ferner erreicht werden, dass die mechanischen Eigenschaften und die chemischen Inhaltsstoffe, die bei neuen Stoffen potentiell auf ihre Verträglichkeit getestet werden müssten, konstant bleiben.
Ein weiterer großer Vorteil ist die Möglichkeit mit den porösen Trägern eine dreidimensionale Struktur für das Gewebe während der Vernetzung vorgeben zu können, die ihre spätere Funktion als Implantat unterstützt. Insbesondere bei Verwendung von offenporigen Polymerträgern bzw. Formbereichen aus Polymer, wie z.B. aus Polycarbonat, sind komplexe Geometrien durch Rapid Prototyping einfach realisierbar. Dies erlaubt erstmals eine an die Klappengeometrie angepasste Formgebung des Gewebes, wodurch völlig neue Klappengeometrien denkbar sind. Durch die dreidimensionale Formgebung des Gewebes ist es außerdem möglich das Öffnungs- und Schließverhalten der Klappenflügel zu optimieren.

Ein weiterer großer Vorteil ist die Möglichkeit zur dreidimensionalen Formgebung des biologischen Klappenteils während der Gewebeprozessierung und nicht erst in einem späteren Schritt durch Schneiden und Nähen. Dadurch entfallen innere Nähte, die nur zur Formgebung und dabei zum Verbinden vieler einzelner Gewebsstücke notwendig sind. Nähte zur Fixierung am Klappenstent sind zwar weiterhin erforderlich, aber durch die innere Stabilität des Gewebestückes kann deren Anzahl reduziert werden. Durch die neuartige Vorformung erweitert sich zudem das Spektrum der geometrisch realisierbaren Klappendesigns. So ist es auch möglich durch eine vorteilhafte Formgebung die Hämodynamik positiv zu beeinflussen. Dies funktioniert insbesondere durch das vorgeschlagene Verfahren, da die Anzahl von Nähten und damit auch von Knoten, die die Hämodynamik beeinflussen, stark reduziert wird. Des Weiteren kann durch die hierin vorgeschlagene Formgebung die Form natürlicher Herzklappen besonders gut nachempfunden werden, was zusätzlich dazu beiträgt eine natürliche Hämodynamik zu generieren. Dadurch kann ein problemloser Blutfluss erreicht werden und der Verschleiß an den Nähten reduziert werden. Bei einer optimierten Hämodynamik wird ferner das Klappenmaterial wie auch das umliegende Gewebe geschont. Durch die Reduktion der Anzahl notwendiger Nähte reduziert sich auch die Anzahl der potentiellen Schwachstellen in Bezug auf die Langzeithaltbarkeit des Klappenimplantats.

Insbesondere können hier vorteilhaft Ausführungsformen angeführt werden, bei denen nach dem vorliegenden Verfahren die Herzklappe aus drei gleichartigen Stücken gefertigt wird. In einer solchen Ausführungsform wird die Klappe lediglich durch drei Senkrechte Nähte ausgebildet, wobei die drei Kommissurlinien in gleicher Weise mechanisch beansprucht werden. Dadurch wird eine deutliche Reduzierung der Nähte erreicht und eine gleichmäßig beanspruchte Klappe. Ferner ist durch die enorme Einsparung von Nähten eine Klappe bevorzugt, die lediglich aus einem Gewebestück gefertigt ist.

Es steht dem vorliegenden Vorschlag nicht entgegen, dass auch weitere Behandlungsschritte an dem zu vernetzenden oder vernetzten Gewebe durchgeführt werden, die so gleich die Eigenschaften des Gewebes wie Gewebegeometrie, Gewebemechanik und Vernetzbarkeit nicht berühren und insbesondere nicht verschlechtern. Hier kann zum Beispiel eine Dezellularisierung wie im Stand der Technik beschrieben, dem hierin vorgeschlagenen Vernetzungsverfahren vorgeschaltet, angeführt werden.

Im Folgenden soll die Erfindung anhand vom Ausführungsbeispielen anhand der Figuren näher erläutert werden. Es zeigen:
- Fig. 1: schematische Ansicht eines Formwerkzeugs zur Dickenreduktion von Gewebeplättchen ohne (Querschnitt oben) und mit (Querschnitt unten) dreidimensionaler Formgebung;
- Fig. 2: schematische Darstellung des unteren Formbereichs bzw. Formmatrix für die Herstellung des kompletten biologischen Gewebes in einem Stück für eine TAVI-Klappe.
- Fig.3: Dickenreduktion durch Einspannung von porcinem Perikardgewebe während der Vernetzung mit 0,6%v/v Glutaraldehyd zwischen porösen Trägern, hier aus Borosilikatglas, mit den Porengrößen: P3: 16-40 µm; P4: 10-16 µm; P5: 4-5.5 µm im Vergleich zu frei vernetztem porcinem Perikardgewebe.
- Fig. 4: zeigt Shrinkage Temperaturen von porcinem Perikardgewebe, welches durch Einspannung zwischen porösen Formbereichen bzw. Trägern (Porengrößen: Por3: 16-40 µm; Por4: 10-16 µm; Por5: 4-5.5 µm) während der Vernetzung mit 0,6%v/v Glutaraldehyd behandelt wurde im Vergleich zu nativem und zu frei vernetztem porcinem Perikardgewebe.

Das hier vorgestellte Verfahren beschreibt dreidimensionale Formgebung von biologischen Geweben, insbesondere während einer gleichzeitigen Dickenreduktion oder -kontrolle des Gewebes (z.B. Perikardgewebe) während der Fixierung mit Glutaraldehyd oder anderen vernetzenden Agentien durch Vernetzung zwischen porösen Formbereichen eines Formwerkzeuges. Durch diese Raumbeschränkung ist es möglich, die Dicke der Gewebe während der Fixierung zu kontrollieren bzw. zu reduzieren und deren räumliche Form zu beeinflussen. Dies hängt damit zusammen, dass während der Fixierung mit reaktiven Agentien durch die resultierenden strukturellen Veränderungen innerhalb des Gewebes sonst eine deutliche Volumenzunahme bis zu einem Faktor zwei zu beobachten ist. Eingesetzt werden formgebende Elemente eines Formwerkzeuges 1, hier als Formbereiche 3 bezeichnet, die z.B. mit Hilfe des 3D-Drucks aus porösem Kunststoff, wie z.B. Polycarbonat, hergestellt werden.

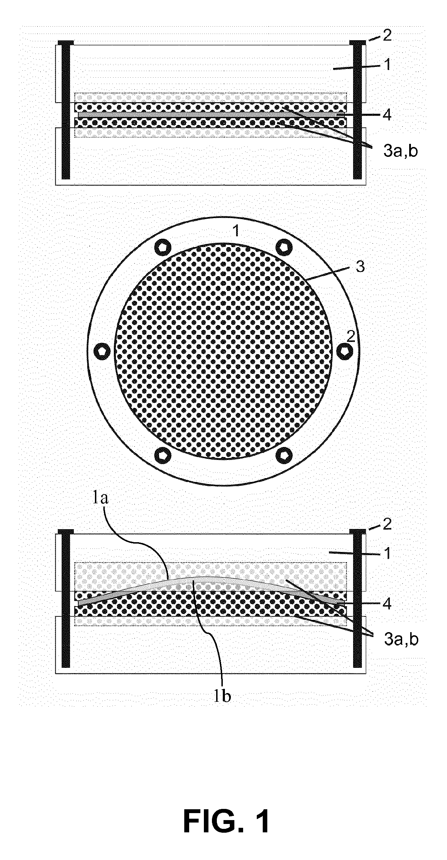

Der verwendete Aufbau bzw. das verwendete Formwerkzeug 1 zur Dickenkontrolle und 3D-Formgebung während der Vernetzung ist schematisch in Fig. 1 (unterer Querschnitt) gezeigt.

Danach weist das Formwerkzeug eine erste 3D-Anlagefläche 1a zur flächigen Anlage an einer Oberseite des Gewebes sowie eine zweite 3D-Anlagefläche 1b zur flächigen Anlage an einer Unterseite des Gewebes 4 auf, wobei das Gewebe 4 zwischen den beiden Anlageflächen 1a, 1b einspannbar ist, so dass es beidseitig an diesen anliegt und dabei mittels eines Vernetzungsmittels zu vernetzen ist, so dass das vernetzte Gewebe 4 nach Entnahme aus dem Formwerkzeug eine 3D-Form sowie ggf. eine vordefinierte Dicke aufweist.

Das Formwerkzeug 1 weist dabei einen oberen Formbereich 3a auf, der jene erste 3D-Anlagefläche 1a ausbildet, sowie einen unteren Formbereich 3b, der jene zweite 3D-Anlagefläche 1b ausbildet. Die Formbereiche 3 sind für das Vernetzungsmittel durchlässig, so dass dieses während der Vernetzung über die Formbereiche zu dem Gewebe gelangen und dieses kontaktieren kann.

Das zu vernetzende Gewebe wird im nativen Zustand zwischen die porösen Formbereiche eingelegt und mit Hilfe einer Fixierung 2 (z.B. durch Klammern oder Schrauben), eng anliegend fixiert.

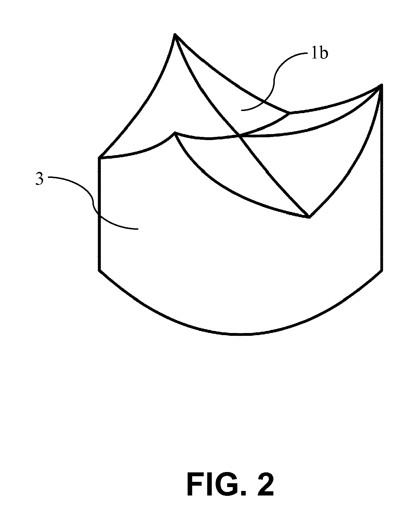

Figur 2 zeigt schematisch den unteren Formbereich bzw. Teil der Formmatrix für das biologische Gewebe einer TAVI-Klappe. Durch Verwendung einer derartigen Matrix ist es möglich den kompletten Gewebeteil einer Klappe in einem Stück herzustellen, welches sich in den Klappenstent einnähen lässt. Die Formgebung an einem Stück erfolgt durch die Verwendung von drei (für jeden Flügel eines; nicht gezeigt) Formstücken, die während der Vernetzung von oben auf das gezeigte Unterteil 3 aufgesetzt werden. Bei dieser Ausführungsform besteht zum Beispiel die gesamte Klappe aus einem einzigen zusammenhängenden Gewebestück, welches die endgültige Form mit nur einer senkrechten Seitennaht erhält. Der obere Rand des Gewebes wird in der erfindungsgemäßen Lösung zwischen den drei oberen Formteilen herausgeführt und nach der Vernetzung abgeschnitten (z.B. manuell mit einer chirurgischen Schere oder in einem Laserschneider für 3D-Objekte). Dies vermeidet Faltenbildung während der Formgebung. Des Weiteren ist es möglich das Gewebe durch definiertes Spannen geometrisch zu homogenisieren. Wie bereits angemerkt resultiert vorteilhafterweise durch das hierin vorgeschlagene Verfahren ein (eins) vorgeformtes Gewebestück, welches nur noch eine senkrechte Seitennaht und das Vernähen in den Klappenstent erfordert. Durch das vorgeschlagene Verfahren und die daraus resultierenden Gewebsstücke werden Herstellungskosten und -zeit signifikant reduziert.

### Beispiel:

Im Folgenden ist ein exemplarischer Prozess für die Dickenkontrolle und dreidimensionale Formgebung von porcinem Perikardgewebe während der Vernetzung mit Glutaraldehyd beschrieben.

Zunächst wird ein Herzbeutel vom Schwein am Schlachthof frisch entnommen und für 2h bei 4°C in NaCl (0,9%w/v) mit Penicillin/Streptomycin gelagert.

Sodann wird das Perikardgewebe feucht in NaCl (0,9%w/v) präpariert, wobei FettBindegewebe entfernt werden und das Gewebe zugeschnitten wird.

Hiernach wird das Gewebe in 100ml NaCl (0,9%w/v) unter leichter Bewegung gespült.

Sodann wird das Perikardgewebe zwischen den porösen dreidimensionalen Formbereichen (Polycarbonat) eingespannt, wobei die porösen Formbereiche mit NaCl (0,9%w/v) gesättigt sind.

Anschließend erfolgt eine Vernetzung des eingespannten Gewebes in 100ml Glutaraldehyd-Lösung (0,6%v/v in DPBS ohne Ca/Mg) für 48 Stunden bei 4°C.

Hieran schließt sich eine Vernetzung in 100ml Glutaraldehyd-Lösung (0,6%v/v in DPBS ohne Ca/Mg) an, und zwar für 14 Tage bei Raumtemperatur (typisch 20°C bis 25°C), wobei alle zwei Tage die Glutaraldehyd-Lösung durch eine frische Glutaraldehyd-Lösung ausgetauscht wird.

Das solchermaßen vernetzte Perikardgewebe wird aus den porösen dreidimensionalen Formbereichen bzw. Polycarbonat-Trägern entnommen und wird in 100ml NaCl (0,9%w/v) bei 37°C unter leichter Bewegung gespült, und zwar 6x für 10 Minuten.

Hiernach kann das Gewebe gelagert (vorzugsweise in 100ml Glutaraldehyd-Lösung (0,6%v/v in DPBS ohne Ca/Mg)) bzw. weiteren Verarbeitungsschritten, wie einem Zuschneiden, zugeführt werden.

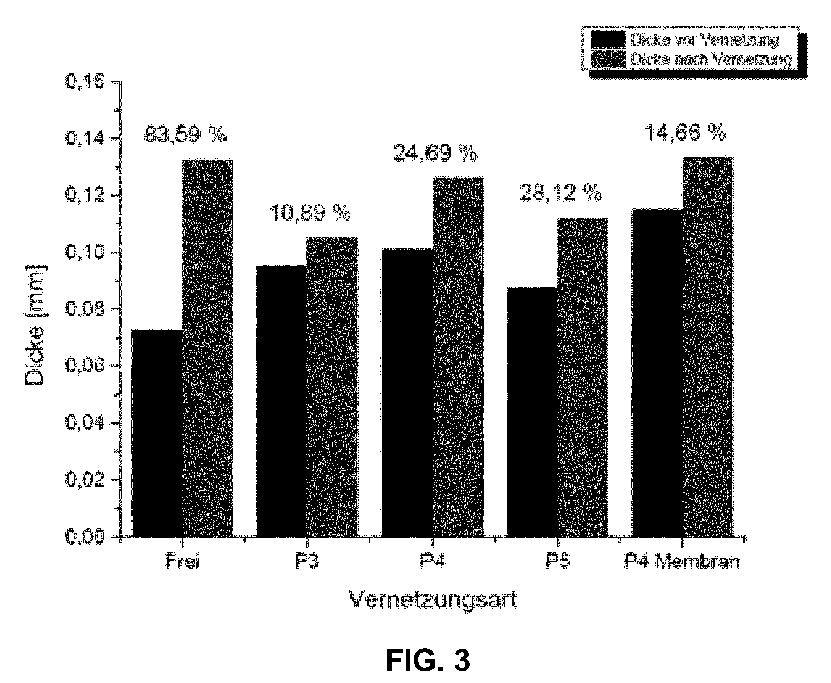

Fig. 3 zeigt die absoluten Dicken in mm und die Dickenzunahme in % relativ zum unvernetzten Ausgangszustand für verschiedene poröse Formbereichsmaterialien (Porengrößen: P3: 16-40 µm; P4: 10-16 µm; P5: 4-5.5 µm) und für frei vernetztes porcines Perikardgewebe (n = 10). Die Vernetzung ohne Formwerkzeug führt zu einer Dickenzunahme von etwa 84%, was eine Folge des Einbaus zusätzlicher chemischer Bindungen und der resultierenden Strukturänderungen der Kollagenfasern ist. Durch Einspannung zwischen den Formbereichen wird diese Dickenzunahme signifikant reduziert. Und dies ohne dass sich der Vernetzungsgrad des Gewebes, gemessen über die Shrinkage Temperatur, von einer Vernetzung ohne Trägermaterialen unterscheidet.

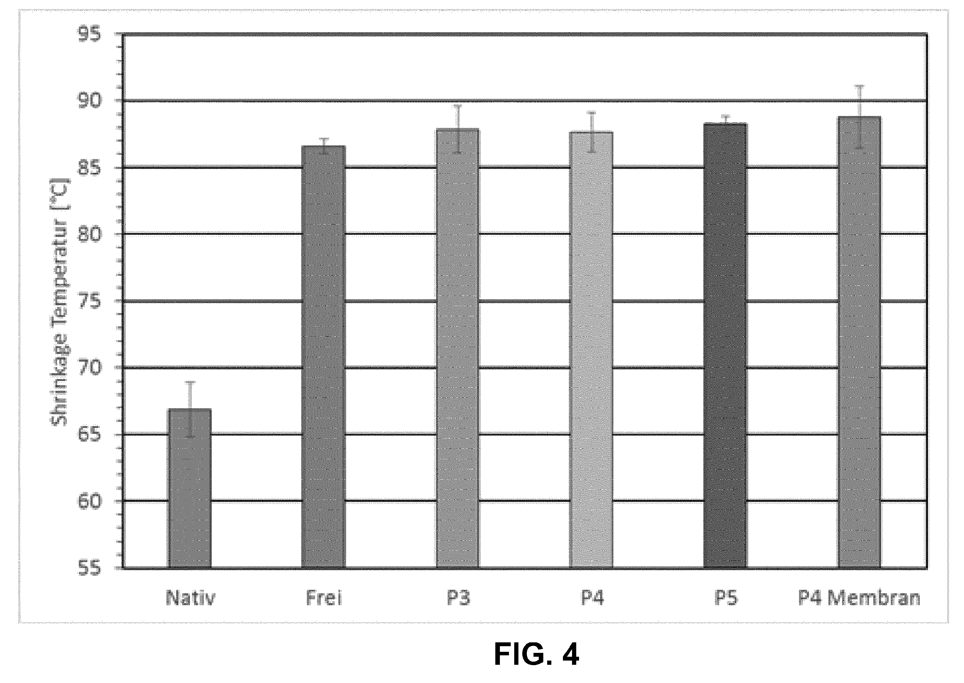

Fig. 4 zeigt die Shrinkage Temperaturen der Gewebe aus Fig. 3, wobei insgesamt jeweils zehn Probenstücke vermessen wurden, die aus allen Bereichen der vernetzten Gewebefläche entnommen wurden. Es ist deutlich die Zunahme der Shrinkage Temperatur infolge der Vernetzung nachweisbar, was die gleichmäßige Vernetzung aller Gewebe zeigt, d.h. die Zugänglichkeit des Glutaraldehyd zum Gewebe ist durch die porösen Formbereiche gewährleistet.

## Patentansprüche

1. Verfahren zum Aufprägen einer 3D-Form auf ein biologisches Gewebe (4), insbesondere Perikardgewebe (4), während der Vernetzung des Gewebes (4), unter Verwendung eines Formwerkzeuges (1), das eine erste 3D-Anlagefläche (1a) zur flächigen Anlage an einer Oberseite des Gewebes (4) sowie eine zweite 3D-Anlagefläche (1b) zur flächigen Anlage an einer Unterseite des Gewebes (4) aufweist, wobei das Gewebe (4) zwischen den beiden Anlageflächen (1a, 1b) angeordnet wird, so dass es beidseitig an diesen anliegt und dabei mittels eines Vernetzungsmittels vernetzt wird, so dass das vernetzte Gewebe (4) nach Entnahme aus dem Formwerkzeug eine 3D-Form aufweist.

2. Verfahren nach Anspruch 1, **dadurch gekennzeichnet, dass** die Dicke des vernetzten Gewebes durch einen Abstand der beiden 3D-Anlageflächen (1a, 1b) zueinander auf einen maximalen Wert begrenzt wird.

3. Verfahren nach Anspruch 1 oder 2, **dadurch gekennzeichnet, dass** das Formwerkzeug (1) einen oberen Formbereich (3) aufweist, der jene erste 3D-Anlagefläche (1a) ausbildet, und dass das Formwerkzeug (1) einen unteren Formbereich (3) aufweist, der jene zweite 3D-Anlagefläche (1b) ausbildet.

4. Verfahren nach Anspruch 3, **dadurch gekennzeichnet, dass** die Formbereiche (3) für das Vernetzungsmittel durchlässig sind, so dass dieses während der Vernetzung über die Formbereiche (3) zu dem Gewebe (4) gelangen und dieses kontaktieren kann.

5. Verfahren nach einem der vorangegangenen Ansprüche, **dadurch gekennzeichnet, dass** das Vernetzungsmittel eine Glutaraldehyd-haltige Lösung ist, die vorzugsweise 0,01%v/v bis 2%v/v Glutaraldehyd aufweist, vorzugsweise in DPBS ohne Ca/Mg.

6. Verfahren nach einem der vorangegangenen Ansprüche, **dadurch gekennzeichnet, dass** das Vernetzungsmittel Glutaraldehyd, Carbodiimid, Formaldehyd, Glutaraldehyd-Acetale, Acyl-Azide, Cyanimid, Genepin, Tannin, Pentagalloyl-Glukose, Phytat, Proanthocyanidin, Reuterin und/oder Epoxidverbindungen enthält.

7. Verfahren nach einem der vorhergehenden Ansprüche, **dadurch gekennzeichnet, dass** das im Formwerkzeug (1) angeordnete Gewebe (4) dem Vernetzungsmittel 1 bis 3 Tage, vorzugsweise 2 Tage, bei 2°C bis 10°C, vorzugsweise bei 4°C, ausgesetzt wird.

8. Verfahren nach Anspruch 7, **dadurch gekennzeichnet, dass** das Gewebe (4) anschließend dem Vernetzungsmittel 10 bis 18 Tage, vorzugsweise 14 Tage, vorzugsweise bei Raumtemperatur, ausgesetzt wird, wobei bevorzugt das Vernetzungsmittel alle 1 bis 3 Tage, vorzugsweise alle 2 Tage, ausgewechselt wird.

9. Implantat, **dadurch gekennzeichnet, dass** es Gewebe (4) aufweist, welchem eine 3D-Form mit einem Verfahren nach einem der Ansprüchen 1 bis 8 aufgeprägt wurde.

10. Implantat, nach Anspruch 9, **dadurch gekennzeichnet, dass** das Implantat eine Herzklappenprothese ist, welche eine künstliche Herzklappe aus dem Gewebe (4) aufweist, welches an einen expandierbaren oder selbstexpandierenden und per Katheter implantierbaren Grundkörper befestigt, bevorzugt vernäht, ist.
